# EUROPEAN PATENT APPLICATION

(11) **EP 1 570 862 A1**
(43) Date of publication of application: **07.09.2005**
(21) Application number: 04004227.7
(22) Date of filing: 25.02.2004
(51) Int. Cl.: A61K 47/48, A61K 31/137, A61K 31/445

(54) **Highly soluble binary cyclodextrin inclusion complexes**

(71) Applicant: JPM - The Jordanian Pharmaceutical Manufacturing Co. Ltd., 11710 Naor (JO)
(72) Inventor: Badwan, Adnan, Dr., 11185 Amman (JO); Omari, Mahmoud, M., H., Dr., 11821 Amman (JO); Zughul, M., B., 11192 Jabal Al-Hussain (JO); Davies, Eric, Dr., Carms., SA 17 4UF (GB)
(74) Representative: Goddar, Heinz J.

(57) **Abstract**

The present invention relates to highly soluble binary cyclodextrin inclusion complexes consisting of an amphoteric compound and a cyclodextrin. Said complexes are easily water-soluble with extremely high concentration both of the compound and cyclodextrin.

## Description

The present invention relates to highly soluble binary cyclodextrin inclusion complexes consisting of an amphoteric drug and a cyclodextrin.

The term "amphoteric drug" refers to organic compounds bearing hydrophobic groups, nitrogen basic and carboxylic, sulphonic, sulfuric or phenolic groups. The basic and acid moieties may be aliphatic, cyclic or aromatic. Fexofenadine is one example of these amphoteric drugs and has the following formula.

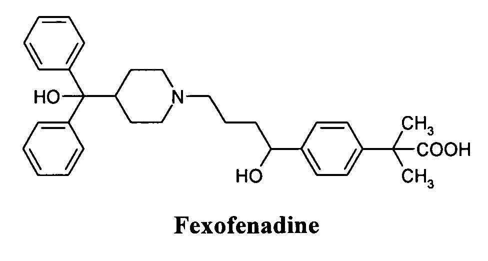

A lot of drugs suffer from low water solubility that often hampers their application. To enhance their solubility, basic drugs usually converted to salts with inorganic and organic acids, while acidic drugs converted to alkali salts such as sodium or potassium salts. However, sometimes even the salts are poorly soluble, or by other reasons (e.g. stability, process) a conventional salt formation is inadequate.

The parent cyclodextrins α-, β-, γ-cyclodextrins and their derivatives are widely used to improve the solubility of water insoluble compounds through inclusion complexation (dissolution and bioavailability improvements). Also they are used to enhance the thermal stability, to reduce volatility, and to resist oxidation, hydrolysis and degradation of compounds in solution.

The parent cyclodextrins vary in their water solubility, while γ-cyclodextrin is the most water soluble (23.2 g/100 ml at 25°C), β-cyclodextrin is the least soluble (1.8 g/100 ml at 25°C). Solubility of β-cyclodextrin can be enhanced by preparing different derivatives of this polymer such as hydroxypropyl-β-cyclodextrins and sulfobutylether-β-cyclodextrins. These polymers efficiency in solublization of the water insoluble compounds is increased with their solubility increase in the system. Among all CDs used, β-cyclodextrin is the most utilized in pharmaceutical investigations due to its abundance and cavity diameter. It is sparingly soluble in water. This limited aqueous solubility is attributed to a relatively high crystal lattice energy and the existence of intramolecular hydrogen bonding between the hydroxyl groups of β-cyclodextrin.

It was reported early by Ventura et al¹. and Badwan et al². that presence of organic and inorganic acids in ternary mixtures enhanced the solubilizing effect of β-cyclodextrin on basic drugs. These complexes offer higher solubility for both substrate and β-cyclodextrin exceeding their individual maximum solubility, the solubility of the hydrophilic β-cyclodextrin may be increased up to more than 10 times.

Kellawy et al.³ were first to give an indicative remark by phase solubility diagram about the increase in solubility of β-cyclodextrin beyond its solubility limit when complexing with the amphoteric drug salbutamol.

A first aspect of the present invention therefore relates to binary inclusion complexes fundamentally consisting of an amphoteric drug and a cyclodextrin.

A second aspect of the present invention relates to the preparation of complexes with a cyclodextrin, and particularly with β-cyclodextrin, with the purpose of increasing water solubility of the cyclodextrin itself.

Up to now, neither highly binary inclusion complexes containing a cyclodextrin and an amphoteric drug have been described, nor a so high improvement of solubility of an active ingredient and cyclodextrin in a binary inclusion complexation has been obtained.

The simultaneous large solubility enhancement for β-cyclodextrin at least up to here disclosed extent is also a new and surprising observation in a binary system.

A further object of this invention is a general method for preparation of amphoteric drug/β-cyclodextrin complex following method reported early by Ventura et al¹ by removal of distilled water from the highly supersaturated solution of the two components in distilled water. According to the present invention, binary complex preparation comprises the following steps:
1) Suspension in distilled water of suitable quantities of drug and cyclodextrin.
2) Dissolving by stirring or/and sonication to obtain a clear or slightly opalescent solution.
3) Filtration of the solution obtained in step 2 by using a suitable system to obtain a clear solution.
4) Dehydration of the solution obtained in step 3 by using dehydration methods such as freeze-drying, spray-drying, vacuum drying at moderate temperature or similar.

The same method can be used in order to prepare complexes with α-CD, γ-CD, hydroxypropyl-β-CD, dimethyl-β-CD, sulfobutylether-β-CD, or other cyclodextrin derivatives.

Even more, the supersaturated solution with improved storage stability, which can be used in oral and parenteral formulations, are obtained by using these entirely more soluble cyclodextrins and β-cyclodextrin derivatives.

Amine containing- phenols, carboxylic, sulphonic, or sulfuric or phenolic acids of different chemical and therapeutic classes, have been selected as test molecules. Particularly surprising results have been obtained with diphenylmethane and phenol derivatives.

Nevertheless, it is evident that the present invention is applicable to every amphoteric compound.

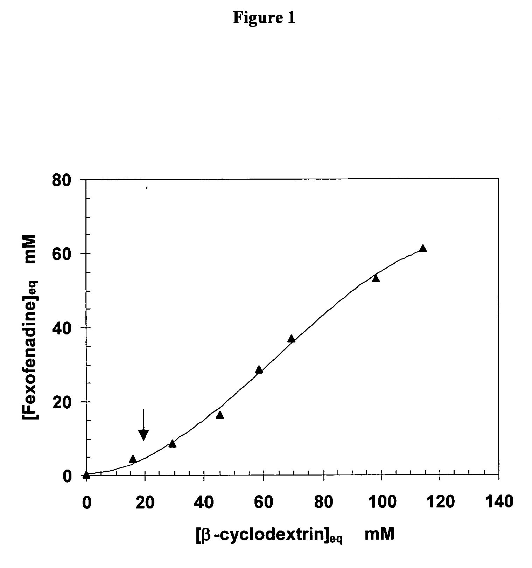

The invention shall now be further described in the following examples, without being limited thereto. The accompanying Figure 1 shows a phase solubility diagram of fexofenadine/β-cyclodextrin system at 30°C. Arrow represents the optimal solubility of β-cyclodextrin.

### Example 1

### Mutual solubility enhancement of fexofenadine/ βcyclodextrin in water:

To illustrate the mutual solubility enhancement for both drug and β-cyclodextrin in water, excess amounts of fexofenadine and β-cyclodextrin (well beyond the optimal solubility of β-cyclodextrin corresponding to 20 mM at 30°C). The samples were mechanically shaken in a thermostatic bath shaker (~ 200 rpm) to attain thermal equilibrium, an aliquot was centrifuged (when necessary) and filtered using a 0.45µm filter (cellulose acetate or cellulose nitrate, Advantec MFS Inc., Duplin, USA). The drug assay was conducted using first derivative spectrophotometry at about 270 nm. β-cyclodextrin assay was performed by optical rotation (α) measurements on a Polartronic D at 25°C using a 1 dm cell.

Figure 1 shows the phase solubility diagram obtained for fexofenadine in water. The samples were shaken for different time intervals. The corresponding [β-cyclodextrin]_{eq} value was 115 mM ( 6 times the optimal solubility of β-cyclodextrin, which indicate significant synergism without supersaturation. The equilibrium time did not exceed 1 day and no any precipitate was observed after about 1 month of mechanical shaking, in addition the filtrate solutions monitored for 1 month did not show any precipitate.

### Example 2

### Preparation of fexofenadine/β-cyclodextrin soluble complex:

Mixtures of fexofenadine and β-cyclodextrin (3 mmol: 6 mmol) were dissolved in 50 ml of water by mechanical shaking and then the solution obtained was freeze-dried. The fexofenadine content of the solid complex was about 18%. DCS analysis confirmed the formation of the complex.

### References

1- Ventura P, Chiesi P, Pasini M, Redenti E, Szejtle J, Vikmon M. 1995. Highly Soluble Multicomponent Inclusion Complexes Containing a Base Type Drug, an Acid and a Cyclodextrin. Chiesi Farmaceutici S.P.A. Company (Italy). US Patent No. US 5,855,916.
2- Badwan AA, Abu-Malooh A, Haddadin M, Ibrahim H. 1997. Method for Solubilizing Drugs Using Cyclodextrins and Carboxylic Acids. Arab company for Drug Industries and Medical Applicances (ACDIMA, Jordan). US Patent No. 5,646,131 A 1997-07-08.
3- Cabral HM, Hadgraft MJ, Kellawy IW. 1990. Studies of Cyclodextrin Inclusion Complexes. I. The Salbutamol-Cyclodextrin Complex as Studied by Phase Solubility and DSC. *International Journal of Pharmaceutics*. **63**: 259-266.

## Claims

1. Binary inclusion complexes **characterized in that** an amphoteric drug (guest molecule) and a cyclodextrin are present.

2. Inclusion complexes according to claim 1, **characterized in that** both the guest molecule and the cyclodextrin have a very water solubility.

3. Inclusion complexes according to claim 1 or 2, **characterized in that** cyclodextrin is selected from the group consisting of α-CD, γ-CD, hydroxypropyl-β-CD, dimethyl-β-CD, sulfobutylether-β-CD, or other cyclodextrin derivatives.

4. Inclusion complexes according to any of claims 1 to 3, **characterized in that** the amphoteric drug is selected from the group consisting of fexofenadine as an example of amine containing- carboxylic, salbutamol as amine containing-phenol.

5. A pharmaceutical composition, comprising a binary inclusion complex of any of claims 1 to 4.

6. A method for preparing of binary inclusion complexes of the preceding claims comprising the following steps:
a) Suspension in distilled water of suitable quantities of drug and cyclodextrin,
b) Dissolving by stirring or/and sonication to obtain a clear or slightly opalescent solution,
c) Filtration of the solution obtained in step b by using a suitable system to obtain a clear solution, and
d) Dehydration of the solution obtained in step c.
